Europäisches Patentamt

(19) European Patent Office

Office européen des brevets

(11) Publication number: **0 213 750 B1**

(12) **EUROPEAN PATENT SPECIFICATION**

(45) Date of publication of patent specification:
02.01.91 Bulletin 91/01

(51) Int. Cl.⁵: **A61M 29/02**

(21) Application number: 86305875.6

(22) Date of filing: 30.07.86

(54) Self-venting balloon dilatation catheter.

(30) Priority: 30.07.85 US 760637

(43) Date of publication of application:
11.03.87 Bulletin 87/11

(45) Publication of the grant of the patent:
02.01.91 Bulletin 91/01

(84) Designated Contracting States:
AT CH DE FR GB IT LI NL

(56) References cited:
FR-A- 1 525 843
US-A- 4 323 071

(73) Proprietor: ADVANCED CARDIOVASCULAR
SYSTEMS, INC.
1395 Charleston Road
Mountain View, CA 94039-7101 (US)

(72) Inventor: Powell, Phillip E.
750 Sterling Road Apartment No. 94
Mountain View California 94043 (US)

(74) Representative: Bizley, Richard Edward et al
BOULT, WADE & TENNANT 27 Furnival Street
London EC4A 1PQ (GB)

EP 0 213 750 B1

## Description

This invention relates to a self-venting balloon dilatation catheter.

In utilising balloon dilatation catheters, the balloon must be filled with a liquid. In the filling of the balloon, the air which is within the balloon should be expelled from the balloon, but the air is compressible. In the past expelling has been accomplished by successively aspirating the balloon with fluid. The air is withdrawn during the repeated evacuation. This has a disadvantage in that ensuring complete removal of all the air can be difficult.

US-A-4323071 (Simpson & Robert) discloses a self-venting balloon dilatation catheter comprising a flexible tubular member with first and second lumens extending therethrough, an inflatable balloon carried by a distal end portion of the tubular member, the first lumen extending through the balloon and not being in communication with the interior of the balloon and the second lumen being in communication with the interior of the balloon, and means for venting air from the interior of the balloon.

In this known construction, the means for venting is a removable tube which extends from the proximal extremity of the catheter into the balloon so that during the time that the liquid is being introduced into the balloon, the air in the balloon is vented through the separate tube.

The use of such a separate tube has the disadvantage that making a dilatation catheter having a very low profile is difficult.

The present invention overcomes such a disadvantage and is characterised in that the venting means-comprises at least one passageway in the flexible tubular member which is less than 0.025mm (0.001 inch) in diameter and extends through the distal extremity of the flexible tubular member for venting air from the interior of the balloon to the exterior of the catheter but inhibiting the escape of liquid from the balloon.

In the accompanying drawings :

Figure 1 is a side elevational view of a balloon dilatation catheter ;

Figure 2 is a cross-sectional view of the distal extremity of the balloon dilatation cathether shown in Figure 1 ;

Figure 3 is a cross-sectional view taken along the line 3-3 of Figure 2 ; and

Figure 4 is a cross-sectional view showing a step in a method which is utilised in manufacturing the balloon dilatation catheter shown in Figures 1 to 3.

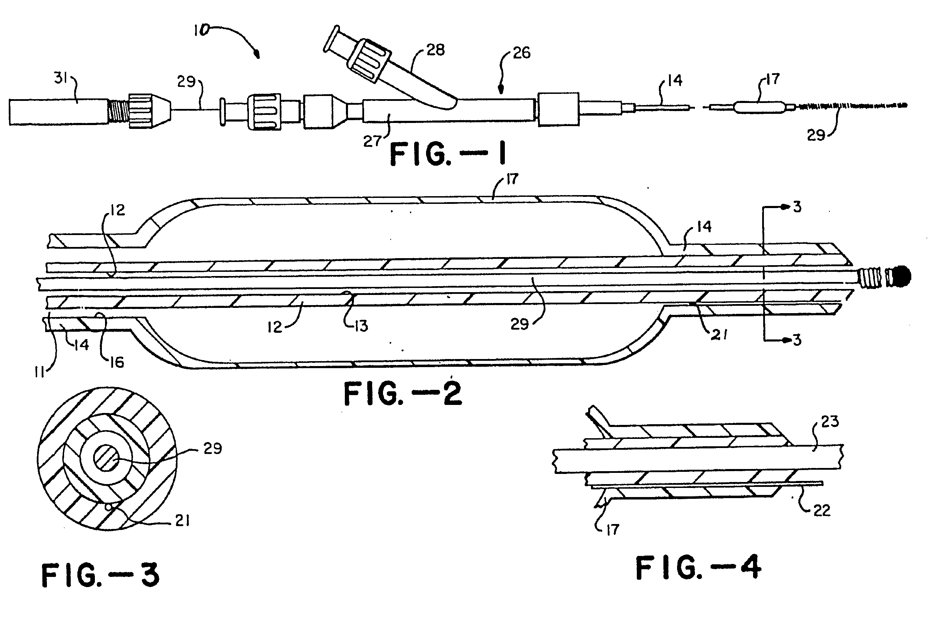

As shown in Figures 1 to 3 of the drawings, a balloon dilatation catheter 10 comprises a tubular member 11, comprising a first tubular element 12 which has a lumen 13 extending therethrough, and a second tubular element 14, which is coaxially disposed on the first tubular element 12, and forms, in conjunction with the first tubular element 12, an annular lumen 16 which extends longitudinally of the first and second tubular elements 12 and 14. An expandable balloon 17 is carried by the second tubular element 14 of the member 11 near the distal portion thereof and has its interior in communication with the lumen 16. The balloon 17 extends co-axially about the first tubular element 12. Although the balloon 17 can be formed as a separate element which has its extremities bonded to the second tubular element 14, it is preferably formed integral with the second tubular element as shown. The tubular elements 12 and 14 are formed of a suitable flexible thermo-plastic material such as a polyolefin or polyvinylchloride.

The distal extremities of the first and second tubular elements 12 and 14 are bonded together in a suitable manner so as to form a liquid-tight seal between them. Typically, this can be accomplished by applying heat to the distal extremity of the second tubular element 14 with a mandrel disposed in the distal extremity of the first tubular element 12 and applying heat to shrink the distal extremity of the-second tubular element 14 onto the first tubular element 12 to form such a seal.

Means is provided in the distal extremity of the first and second tubular elements 12 and 14 for venting air from the balloon 17 while inhibiting the escape of liquid from the balloon 17 and comprises a very small passage 21 which is disposed between the distal extremities of the first and second tubular elements 12 and 14 and which extends from the interior of the balloon 17 to the environment at the distal extremity of the tubular member 11.

The flow passage 21 can be formed in any suitable manner. One method found to be particularly efficacious is described in conjunction with Figure 4. A piece of suitable wire 22 such as tungsten is used because of its good tensile strength. The wire 22 has a diameter which is less than 0.025mm (0.001 inch), for example 13 μm (0.0005 inch). It is coated with silicone. After the wire 22 has been coated with silicone, it is inserted by tweezers between the distal extremities of the first and second tubular elements 12 and 14 prior to the time that the second tubular element 14 is heat shrunk onto the first tubular element 12 as hereinbefore described. As soon as the tungsten wire 22 has been inserted into the distal extremities of the first and second tubular elements 12 and 14, so that it extends into the balloon 17 and out of the distal extremities as shown in Figure 4, a mandrel 23 is inserted into the lumen 13. Heat is then applied to the distal extremity of the second tubular element 14 to cause it to form a shrink fit between it and the distal extremity of the first tubular element 12 and at the same time to shrink down around the wire 22. After the distal extremity of the tubular member 11 has been cooled, the mandrel 23 is removed and the wire 22 pulled out with tweezers leaving the cylindrical flow passage 21 hereinbefore described.

If desired, more than one hole or passage 21 can be provided to make the balloon venting procedure more rapid. Other means can be provided in the distal extremity of the catheter in place of the passage 21 for making the balloon 17 self-venting. For example, braided fibres can be utilised in the distal extremity of the catheter in the same manner as the tungsten wire 22 has been utilised. In such a case, the fibres can be left in place so that the air can flow between the interstices of the braided fibres. Alternatively, one or more hollow fibres can be incorporated into the distal extremity of the catheter. Alternatively or additionally, hydrophobic filter material can be incorporated between the distal extremities of the first and second tubular elements 12 and 14. This filter material is capable of passing air but inhibits the passage of aqueous or hydrophilic liquid from the balloon 17.

The remainder of the balloon dilatation catheter shown in Figure 1 is substantially conventional. A side arm adapter 26 is provided which has a main or central arm 27 and a side arm 28. A guide wire 29 extends through the main or central arm 27 and extends through the lumen 13 of the first tubular element 12 and has a distal extremity extending beyond the distal extremity of the tubular member 11. A fitting 31 is secured to the proximal extremity of the guie wire 29 and is utilised for extending and retracting the guide wire and also for rotating the guide wire.

Use of the self-venting balloon dilatation catheter is now briefly described. The balloon 17 is first inflated outside of the human body by introducing a radiographic contrast liquid through the side arm 28 so that it passes through the annular lumen 16 between the first and second tubular elements 12 and 14 and passes into the balloon 17. The air which is in the balloon is pushed forwardly in the balloon and under the pressure of the radiographic contrast liquid is forced out through the small passage 21 provided between the distal extremities of the first and second tubular elements 12 and 14. By utilising a passsage 21 having a diameter of 13 µm (0.0005 inch), a 2mm diameter balloon having a length of approximately 25mm can be completely vented of air in less than approximately 40 seconds. The size of the passage 21 is such that it inhibits the escape of the radiographic contrast liquid so that very little, if any, of the liquid can escape, even though pressures up to 1.37MPa (200 psi) for the radiographic contrast liquid can be reached within the balloon 17. As soon as the balloon 17 has been inflated with the radiographic contrast liquid and the air has been expelled therefrom through the passage 21, the liquid is withdrawn to deflate the balloon 17. The balloon dilatation catheter is now ready to be inserted into the human body. After the balloon 17 has been positioned in the stenosis in the arterial vessel in the human body, the balloon is again inflated by reintroducing radiographic contrast liquid through the side arm 28 through the lumen 16 and into the balloon 17. Since all of the air has previously been expelled from the balloon 17, the balloon can be readily inflated within the stenosis to its full diameter at the desired pressure, for example, in excess of 0.68 MPa (100 psi) without danger of any significant amount of radiopaque contrast liquid passing through the passage 21. After the opening in the stenosis has been enlarged, the balloon can be deflated and the dilatation catheter can be removed.

In the foregoing there has been described a balloon dilatation catheter which is self-venting and in which the balloon 17 can be inflated to the desired pressure without danger of any significant amount of radiopaque contrast liquid passing through the venting orifice 21 provided in the distal extremity of the balloon dilatation catheter. The venting orifice 21 in the distal extremity of the balloon dilatation catheter is formed in such a manner so that it can be readily incorporated into the manufacturing process for making the balloon dilatation catheters.

## Claims

1. A self-venting balloon dilatation catheter (10) comprising a flexible tubular member (11) with first and second lumens (13,16) extending therethrough, an inflatable balloon (17) carried by a distal and portion of the tubular member (11), the first lumen (13) extending through the balloon (17) and not being in communication with the interior of the balloon 17 and the second lumen (16) being in communication with the interior of the balloon (17), and means for venting air from the interior of the balloon (17), characterised in that the venting means comprises at least one passageway (21) in the flexible tubular member (11) which is less than 0.025mm (0.001 inch) in diameter and extends through the distal extremity of the flexible tubular member (11) for venting air from the interior of the balloon (17) to the exterior of the catheter but inhibiting the escape of liquid from the balloon (17).

2. A dilatation catheter as claimed in claim 1, characterised in that the passageway (21) includes hydrophobic material.

3. A dilatation catheter as in claim 1, or 2, the tubular member (11) comprising a first tubular element (12) having the first lumen (13) extending therethrough, a second tubular element (14) being coaxially disposed aver the wherein first tubular element (12) and forming the second lumen (16), the second annular lumen (16) extending between the first and second tubular elements (12,14), the inflatable balloon (17) being carried by the second tubular element (14) with the distal end thereof being sealed to the first tubular element (12), the passageway (21) passes between the first and second tubular elements (12,14) and is in communication with the second

annular lumen (16).

## Ansprüche

1. Selbstventilierender Ballondilatationskatheter (10), umfassend ein flexibles rohrförmiges Teil (11) mit sich dadurch erstreckenden ersten und zweiten Lumina (13, 16), einen aufpumpbaren Ballon (17), der von einem distalen Endabschnitt des rohrförmigen Teils (11) gehalten ist, wobei das erste Lumen (13) sich durch den Ballon (17) erstreckt und mit dem Inneren des Ballons (17) nicht in Verbindung steht und das zweite Lumen (16) mit dem Inneren des Ballons (17) in Verbindung steht, und ein Mittel zum Ventilieren von Luft aus dem Inneren des Ballons (17), **dadurch gekennzeichnet,** daß das Mittel zum Ventilieren wenigstens einen Durchgang (21) in dem flexiblen rohrförmigen Teil (11) aufweist, dessen Durchmesser geringer als 0,025 mm (0,001 inch) ist und der sich durch das distale Ende des flexiblen rohrförmigen Teils (11) erstreckt, um Luft von dem Inneren des Ballons (17) zu der Außenseite des Katheters zu ventilieren, jedoch das Entweichen von Flüssigkeit aus dem Ballon (17) zu verhindern.

2. Dilatationskatheter nach Anspruch 1 **dadurch gekennzeichnet,** daß der Durchgang (21) hydrophobes Material enthält.

3. Dilatationskatheter nach Anspruch 1 oder 2, bei dem das rohrförmige Teil (11) ein erstes rohrförmiges Element (12) aufweist, durch das sich das erste Lumen (13) erstreckt, ein zweites rohrförmiges Element (14) koaxial über dem ersten rohrförmigen Element (12) angeordnet ist und das zweite Lumen (16) bildet, das zweite ringförmige Lumen (16) sich zwischen den ersten und zweiten rohrförmigen Elementen (12, 14) erstreckt, und der aufpumpbare Ballon (17) von dem zweiten rohrförmigen Element (14) gehalten ist, dessen distales Ende zu dem ersten rohrförmigen Element (12) abgedichtet ist, wobei der Durchgang (21) zwischen den ersten und zweiten rohrförmigen Elementen (12,14) verläuft und mit dem zweiten ringförmigen Lumen (16) in Verbindung steht.

## Revendications

1- Cathéter de dilatation à ballonnet à purge automatique (10) comprenant un organe tubulaire flexible (11) traversé par des première et deuxième chambres (13,16), un ballonnet gonflable (17) porté par une partie d'extrémité distale de l'organe tubulaire (11), la première chambre (13) s'étendant à travers le ballonnet (17) et n'étant pas en contact avec l'intérieur du ballonnet (17) et la deuxième chambre (16) étant en communication avec l'intérieur du ballonnet (17) et des moyens permettant de purger l'air de l'intérieur du ballonnet (17), caractérisé en ce que les moyens de purge comprennent au moins un passage (21) dans l'organe tubulaire flexible (11) dont le diamètre est inférieur à 0,025 mm et qui s'étend à travers l'extrémité distale de l'organe tubulaire flexible (11) pour purger l'air de l'intérieur du ballonnet (17) à l'extérieur du cathéter mais empêcher la fuite de liquide hors du ballonnet (17).

2- Cathéter de dilatation selon la revendication 1, caractérisé en ce que le passage (21) comprend un matériau hydrophobe.

3- Cathéter de dilatation selon la revendication 1 ou 2, caractérisé en ce que l'organe tubulaire (11) comprend un premier élément tubulaire (12) traversé par la première chambre (13), un second élément tubulaire (14) placé coaxialement autour du premier élément tubulaire (12) et formant la deuxième chambre annulaire (16), celle-ci s'étendant entre les premier et deuxième éléments tubulaires (12,14), le ballonnet gonflable (17) étant porté par le deuxième élément tubulaire (14) et son extrémité distale étant scellée au premier élément tubulaire (12), le passage (21) passant entre les premier et deuxième éléments tubulaires (12,14) et étant en communication avec la deuxième chambre annulaire (16).

FIG. — 1

FIG. — 2

FIG. — 3

FIG. — 4

EP 0 213 750 B1